# FASCICULE DE BREVET EUROPEEN

(11) **EP 0 850 311 B1**
(45) Date de publication et mention de la délivrance du brevet: **12.03.2003**
(21) Numéro de dépôt: 96925797.1
(22) Date de dépôt: 17.07.1996
(51) Int. Cl.: C12N 15/82, C12N 15/29, C12N 1/21, C12N 5/10, A01H 5/00

(54) **SEQUENCE ADN ISOLEE POUVANT SERVIR DE ZONE DE REGULATION DANS UN GENE CHIMERE UTILISABLE POUR LA TRANSFORMATION DES PLANTES**
ISOLIERTE DNA-SEQUENZ, DIE ALS REGULATIONSREGION IN EIMEM CHIMÄREN GEN DIENEN KANN, DAS FÜR DIE TRANSFORMATION VON PFLANZEN BRAUCHBAR IST
ISOLATED DNA SEQUENCE FOR USE AS A REGULATOR REGION IN A CHIMERIC GENE USEFUL FOR TRANSFORMING PLANTS

(30) Priorité: 19.07.1995 FR 9508980
(43) Date de publication de la demande: 01.07.1998
(73) Titulaire: Bayer CropScience S.A., 69009 Lyon (FR)
(72) Inventeur: DEROSE, Richard, 69009 Lyon (FR); CHAUBET, Nicole, 67800 Strasbourg (FR); GIGOT, Claude, 67800 Strasbourg (FR)
(86) Numéro de dépôt international: FR9601109
(87) Numéro de publication internationale: WO97004114

(56) Documents cités:
- EP-A- 0 507 698
- EP-A- 0 508 909
- EP-A- 0 652 286
- WO-A-95/06128
- PLANT MOLECULAR BIOLOGY, vol. 21, pages 595-605, XP002023307 SUNDAS, A., ET AL.: "cDNA sequence and expression of an intron-containing histone H2A gene from Norway spruce, Picea abies"
- JOURNAL OF MOLECULAR BIOLOGY, vol. 225, pages 569-574, XP002023308 CHAUBET, N., ET AL.: "Genes encoding a histone H3.3-like variant in Arabidopsis contain intervening sequences"
- NUCLEIC ACIDS RESEARCH, vol. 18, no. 23, pages 6767-6770, XP002023309 TANAKA, A., ET AL.: "Enhancement of foreign gene expression by a dicot intron in rice but not in tobacco is correlated with an increased level of mRNA and an efficient splicing of the intron"
- PROGRESS IN NUCLEIC ACID RESEARCH AND MOLECULAR BIOLOGY, vol. 42, pages 229-257, XP002023310 SINIBALDI, R.M., ET AL.: "Intron splicing and intron-mediated enhanced expression in monocots"
- NUCLEIC ACIDS RESEARCH, vol. 14, no. 22, pages 8845-8862, XP002023311 SEILER-TUYNS, A., ET AL.: "A chimeric mouse histone H4 gene containing either an intron or poly(a)addition signal behaves like a basal histone"

## Description

La présente invention concerne l'utilisation de zones de régulation isolées de gènes transcrits de plantes, de nouveaux gènes chimères les contenant et leur utilisation pour la transformation des plantes.

De nombreux caractères phénotypiques associés à l'expression d'un ou quelques éléments géniques peuvent être intégrés dans le génome de plantes et conférer ainsi à ces plantes transgéniques des propriétés agronomiques avantageuses. De façon non extensive on peut citer: les résistances à des agents pathogènes des cultures, la résistance à des produits phytosanitaires phytotoxiques, la production de substances à intérêt alimentaire ou pharmacologique. En plus de l'isolement et la caractérisation des éléments géniques codant pour ces différents caractères, une expression appropriée doit être assurée. Cette expression appropriée peut se situer aussi bien au niveau qualitatif que quantitatif. Au niveau qualitatif, par exemple spatial: expression préférentielle dans un tissu particulier, ou temporel: expression inductible. Au niveau quantitatif par la quantité accumulée du produit d'expression du gène introduit. Cette expression appropriée dépend pour une large part de la présence d'éléments géniques de régulation associés aux transgènes, en particulier pour ce qui concerne les éléments quantitatifs et qualitatifs. Parmi les éléments primordiaux assurant cette régulation appropriée, l'utilisation de zones promotrices homologues ou hétérologues, simples ou combinées a été largement décrite dans la littérature scientifique. L'utilisation de zone de régulation en aval du transgène ont été utilisées a seule fin de mettre une borne permettant d'arrêter le processus de transcription du transgène, sans présupposé quant à leur rôle sur la qualité ou la quantité de l'expression du transgène.

La présente invention concerne l'utilisation d'introns comme zone de régulation isolé de gènes de plantes, de nouveaux gènes chimères les contenant et leur utilisation pour la transformation des plantes. Elle concerne plus particulièrement une séquence d'ADN isolée, pouvant servir de zone de régulation dans un gène chimère utilisable pour la transformation des plantes et permettant l'expression du produit de traduction du gène chimère dans les régions de la plante en croissance rapide, caractérisée en ce qu'elle comprendau moins un intron correspondant au premier intron (intron 1) de la région 5' non-codante d'un gène d'histone végétale, de préférence un gène d'histone végétale du type H3.3. Selon un mode de réalisation particulier, l'invention concerne également l'utilisation conjointe d'une séquence ADN telle que décrite ci-dessus comme zone de régulation et de promoteurs isolés d'un même gène végétal. L'invention permet l'expression appropriée à la fois quantitative et qualitative des transgènes sous le contrôle de ces éléments de régulation génique. Cette expression appropriée obtenue par l'utilisation de la présente invention peut concerner des caractères tels que: la résistance à des agents pathogènes des cultures, la résistance à des produits phytosanitaires phytotoxiques, la production de substances à intérêt alimentaire ou pharmacologique. En outre, elle est particulièrement adaptée pour conférer aux plantes transgéniques une tolérance accrue à des herbicides par une expression préférentielle, qualitative et quantitative, du produit d'expression de gènes chimères codant des enzymes de résistance herbicide dans les régions de la plante en croissance rapide. Selon un mode de réalisation particulier de l'invention, une expression appropriée du gène de résistance herbicide est obtenue par utilisation conjointe d'un élément de régulation promoteur et d'au moins un intron 1 de la région 5' non codante d'un gène d'histone végétale de type H3.3comme zone de régulation. Un tel profil d'expression peut être obtenu pour tous les caractères présentant un intérêt, tels que décrit ci-dessus, lorsque les gènes codant ces caractères contiennent au moins les éléments de régulation utilisés pour conférer une tolérance herbicide accrue. La présente invention concerne également les cellules végétales transformées à l'aide de ces gènes et les plantes transformées régénérées à partir de ces cellules ainsi que les plantes issues de croisements utilisant ces plantes transformées.

Le gène d'histone, dont est issu la séquence ADN selon l'invention, provient d'une plante monocotylédone telle que par exemple le blé, le maïs ou le riz ou de préférence d'une plante dicotylédone telle que par exemple la luzerne, le tournesol, le soja, le colza ou de préférence *Arabidopsis thaliana*. Selon un mode particulier de réalisation de l'invention, la séquence ADN selon l'invention est sélectionnée parmi le groupe consistant en SEQ ID NO:6 et SEQ ID NO:7.

Selon un mode particulier de réalisation de l'invention, la séquence ADN selon l'invention comprend deux introns 1 de la région 5' non-codante d'un gène d'histone végétale du type H3.3 associés, lesdits introns 1 étant identiques ou différents.

Parmi les produits phytosanitaires utilisés pour la protection des cultures, les produits systémiques sont caractérisés en ce qu'ils sont véhiculés dans la plante après application et, pour certain d'entre eux, s'accumulent dans les parties en croissance rapide, notamment les apex caulinaires et racinaires, provoquant, dans le cas des herbicides, l'altération, jusqu'à la destruction, des plantes sensibles. Pour certain des herbicides présentant ce type de comportement, le mode d'action primaire est connu et résulte d'une inactivation d'enzymes caractérisées impliquées dans des voies de biosynthèse de composés nécessaires au bon développement des plantes cibles. Les enzymes cibles de ces produits peuvent être localisées dans différents compartiments subcellulaire et l'observation du mode d'action de produits connus montre le plus souvent une localisation dans le compartiment plastidial.

La tolérance des plantes sensibles à un produit appartenant à ce groupe d'herbicides, et dont la cible primaire est connue, peut-être obtenue par introduction stable dans leur génome d'un gène codant pour l'enzyme cible, d'origine phylogénétique quelconque, mutée ou non quant aux caractéristiques d'inhibition par l'herbicide du produit de l'expression de ce gène. Une autre approche consiste à introduire de façon stable dans le génome des plantes sensibles un gène d'origine phylogénétique quelconque codant pour une enzyme capable de réaliser une métabolisation de l'herbicide en un composé inactif et non toxique pour le développement de la plante. Dans ce dernier cas, il n'est pas nécessaire d'avoir caractérisé la cible de l'herbicide.

Etant donné le mode de distribution et d'accumulation des produits de ce type dans les plantes traitées, il est intéressant de pouvoir exprimer le produit de la traduction de ces gènes de façon à permettre leur expression préférentielle et leur accumulation dans les régions de la plante en croissance rapide où ces produits s'accumulent. De plus, et dans le cas où la cible de ces produits est localisée dans un compartiment cellulaire autre que le cytoplasme, il est intéressant de pouvoir exprimer le produit de la traduction de ces gènes sous forme d'un précurseur contenant une séquence polypeptidique permettant l'adressage de la protéine conférant la tolérance dans le compartiment adéquat, et en particulier dans le compartiment plastidial.

A titre d'exemple illustrant cette approche on peut citer le glyphosate, le sulfosate ou la fosamétine qui sont des herbicides systémiques à large spectre de la famille des phosphonométhylglycines. Ils agissent essentiellement comme inhibiteurs compétitifs vis à vis du PEP (phosphoénolpyruvate) de la 5-énolpyruvylshikimate-3-phosphate synthase (EPSPS, EC 2.5.1.19). Après leur application sur la plante, ils sont véhiculés dans la plante où ils s'accumulent dans les parties en croissance rapide, notamment les apex caulinaires et racinaires, provoquant l'altération, jusqu'à la destruction, des plantes sensibles.

L'EPSPS, cible principale de ces produits est une enzyme de la voie de biosynthèse des acides aminés aromatiques localisée dans le compartiment plastidial. Cette enzyme est codée par un ou des gènes nucléaires et synthétisée sous forme d'un précurseur cytoplasmique puis importée dans les plastes où elle s'accumule sous sa forme mature.

La tolérance des plantes au glyphosate et aux produits de la famille est obtenue par l'introduction stable dans leur génome d'un gène d'EPSPS d'origine végétale ou bactérienne, mutée ou non quant aux caractéristiques d'inhibition par le glyphosate du produit de ce gène. Etant donné le mode d'action du glyphosate, il est intéressant de pouvoir exprimer le produit de la traduction de ce gène de façon à permettre son accumulation importante dans les plastes et de plus, dans les régions de la plante en croissance rapide où les produits s'accumulent.

Il est connu, par exemple d'après le brevet américain 4 535 060, de conférer à une plante une tolérance à un herbicide du type ci-dessus, en particulier la N-phosphonomethylglycine ou glyphosate, par introduction dans le génome des plantes d'un gène codant pour une EPSPS portant au moins une mutation rendant cette enzyme plus résistante à son inhibiteur compétitif (le glyphosate), après localisation de l'enzyme dans le compartiment plastidial. Ces techniques demandent cependant à être améliorées pour une plus grande fiabilité dans l'emploi de ces plantes lors d'un traitement par ces produits dans des conditions agronomiques.

Dans la présente description, on entend par "plante" tout organisme multicellulaire différentié capable de photosynthèse et par "cellule végétale" toute cellule issue d'une plante et pouvant constituer des tissus indifférenciés tels que des cals, ou des tissus différentiés tels que des embryons ou des parties de plantes ou des plantes ou des semences. On entend par gène de tolérance à un herbicide tout gène, d'origine phylogénétique quelconque, codant soit pour l'enzyme cible de l'herbicide, présentant ou non une ou des mutations quant aux caractéristiques d'inhibition par l'herbicide, soit pour une enzyme capable de métaboliser l'herbicide en un composé inactif et non toxique pour la plante. On entend par zones de la plante en croissance rapide, les régions qui sont le siège de multiplications cellulaires importantes, en particulier les régions apicales.

L'invention a également pour objet un gène chimère pour conférer aux plantes une tolérance accrue vis à vis d'un herbicide ayant pour cible l'EPSPS, comprenant, dans le sens de la transcription, une zone promotrice, une zone de régulation, une zone peptide de transit, et une séquence codant pour une enzyme de tolérance aux produits de la famille des phosphonométhylglycines, caractérisé en ce que la zone de régulation comprend une séquence ADN selon l'invention, en particulier une séquence ADN comprenant au moins un intron correspondant au premier intron (intron 1) de la région 5' non-codante d'un gène d'histone végétale de type H3.3. Dans le gène chimère selon l'invention, ladite séquence ADN est positionnée dans une orientation quelconque relativement à son orientation initiale dans le gène duquel elle dérive, et elle permet l'expression préférentielle et l'accumulation de la protéine de tolérance à l'herbicide dans les zones d'accumulation dudit herbicide.

La présente invention concerne également la production de plantes transformées ayant notamment une tolérance accrue à des herbicides s'accumulant dans les zones en croissance rapide, lesdites plantes transformées étant obtenues par régénération de cellules végétales transformées avec un gène chimère selon l'invention, en particulier un gène chimère comportant une séquence codant pour une enzyme de tolérance aux dits herbicides. L'invention a également pour objet la production de plantes transformées ayant une tolérance accrue aux herbicides de la famille des phosphonométhylglycines par régénération de cellules végétales transformées avec un gène chimère selon l'invention, en particulier un gène chimère comportant une séquence codant pour une enzyme de tolérance à ces herbicides. L'invention concerne également des plantes transformées plus tolérantes aux herbicides s'accumulant dans les zones en croissance rapide, ainsi que les plantes issues de croisements utilisant ces plantes transformées. Selon un mode de réalisation de l'invention, la zone peptide de transit comprend, dans le sens de la transcription, au moins un peptide de transit d'un gène végétal codant pour une enzyme à localisation plastidiale, une partie de la séquence de la partie mature N-terminale d'un gène végétal codant pour une enzyme à localisation plastidiale, puis un second peptide de transit d'un gène végétal codant pour une enzyme à localisation plastidiale, de préférence le gène de la petite sous-unité de la ribulose-1,5-bisphosphate carboxylase/oxygénase (RuBisCO) selon la demande de brevet européen/ PCT 508 909. Cette zone caractéristique a comme rôle de permettre le relargage dans le compartiment plastidial d'un polypeptide mature avec une efficacité maximale, de préférence sous forme native.

La séquence codante utilisable dans le gène chimère selon l'invention provient d'un gène de tolérance herbicide d'origine phylogénétique quelconque. Cette séquence peut être notamment celle de l'EPSPS mutée ayant un degré de tolérance au glyphosate.

La zone promotrice selon la demande de brevet européen/ PCT 507 698 peut être d'origine quelconque, sous forme simple ou dupliquée ou combinée d'un gène s'exprimant naturellement dans les plantes, c'est à dire, par exemple bactérienne telle que celle du gène de la nopaline synthase, ou virale telle que celle du transcrit 35S du virus de la mosaïque du chou-fleur, ou de préférence végétale telle que celle de la petite sous-unité de la ribulose-1,5-bisphosphate carboxylase/oxygénase ou de préférence telle que celle d'un gène d'histone végétale et de préférence d'*Arabidopsis thaliana.* On utilise de préférence la zone promotrice d'un gène d'histone du type "H4".

Le gène chimère selon l'invention peut comprendre, en plus des parties essentielles ci-dessus, une zone intermédiaire non traduite (linker) entre la zone promotrice et la zone codante ainsi qu'entre la zone codante et l'intron 1 et qui peut être d'origine phylogénétique quelconque.

Les exemples suivants illustrent à titre limitatif mais non limitatif plusieurs aspects de l'invention: l'isolement des introns selon l'invention et leur utilisation pour la transformation génétiques des plantes ainsi que les qualités améliorées d'expression des gènes hétérologues des plantes transformées à l'aide de ces introns.

### EXEMPLE 1 :

### 1. Obtention d'un fragment d'EPSPS d'Arabidopsis thaliana

a) deux oligonucleotides 20-mers de séquences respectives: 5'- GCTCTGCTCATGTCTGCTCC -3' 5'- GCCCGCCCTTGACAAAGAAA- 3' ont été synthétisés à partir de la séquence d'un gène d'EPSPS d'*Arabidopsis thaliana* (Klee H.J. et al. (1987) Mol. Gen. Genet., 210, 437-442). Ces deux oligonucleotides sont respectivement en position 1523 à 1543 et 1737 à 1717 de la séquence publiée et en orientation convergente.
b) L'ADN total d'*Arabidopsis thaliana* (*var. columbia)* a été obtenu chez Clontech (référence catalogue: 6970-1)
c) On mélange 50 nanogrammes(ng) d'ADN avec 300ng de chacun des oligonucleotides et soumis à 35 cycles d'amplification avec un appareil Perkin-Elmer 9600, dans les conditions de milieu standard pour l'amplification préconisées par le fournisseur. Le fragment de 204pb résultant constitue le fragment d'EPSPS d' *Arabidopsis thaliana*.

### 2. Construction d'une bibliothèque d'un ADNc à partir d'une ligne cellulaire de maïs BMS.

a) On broye 5 g de cellules filtrées dans l'azote liquide et les acides nucléiques totaux extraits selon la méthode décrite par Shure et al. avec les modifications suivantes:
   - le pH du tampon de lyse est ajusté à PH = 9,0;
   - après la précipitation par l'isopropanol, le culot est repris dans l'eau et après dissolution, ajusté à 2,5 M LiCl. Après incubation pendant 12 h à °OC, le culot de la centrifugation de 15 min. à 30000g à 4°C est resolubilisé. L'étape de précipitation par LiCl est alors répétée. Le culot resolublisé constitue la fraction ARN des acides nucléiques totaux.
b) La fraction ARN-polyA+ de la fraction ARN est obtenue par chromatographie sur colonne oligo-dT cellulose telle que décrite dans "Current Protocols in Molecular Biology" .
c) Synthèse d'ADNc double brin à extrémité synthétique EcoRI: elle est réalisée en suivant le protocole du fournisseur des différents réactifs nécessaires à cette synthèse sou forme d'un kit:le "copy kit" de la société In Vitrogen.
   Deux oligonucleotides simples brins et partiellement complémentaires de séquences respectives: 5'- AATTCCCGGG -3' 5'- CCCGGG- 3' (ce dernier étant phosphorylé) sont ligués avec les ADNc double brin à extrémités franches.
   Cette ligation des adaptateurs résulte en la création de sites Sma I accolés aux ADNc double brin et EcoRI sous forme cohésive à chaque extrémité des ADNc double brin.
d) Création de la bibliothèque:
Les ADNc présentant à leurs extrémités les sites artificiels cohésifs EcoRI sont ligués avec le ADNc du bactériophage λgt10 coupé par EcoRI et déphosphorylé selon le protocole du fournisseur Naew England Biolabs.
   Une aliquote de la réaction de ligation a été encapsidée *in vitro* avec des extraits d'encapsidation: Gigapack Gold selon les instructions du fournisseur, cette librairie a été titrée en utilisant la bactérie *E.coli* C600*hfl*. la librairie ainsi obtenue est amplifiée et stockée selon les instructions du même fournisseur et constitue la librairie de ADNc de suspension cellulaire de maïs BMS.

### 3. Criblage de la bibliothèque de ADNc de suspension cellulaire de maïs BMS avec la sonde EPSP d'Arabidopsis thaliana:

Le protocole suivi est celui de "Current Protocols in Molecular Biology" Volumes 1 et 2, Ausubel F.M. et al , publiés par Greene Publishing Associates et Wiley-Interscience (1989)(CPMB). En bref, environ 10⁶ phages recombinants sont étalés sur boîte LB à une densité moyenne de 100 phages /cm^{2.} Les plages de lyses sont répliquées en double sur membrane Hybond N d'Amersham.

L'ADN a été fixé sur les filtres par traitement UV 1600kJ (Stratalinker de Stratagene). Les filtres ont été préhybridés dans: 6xSSC/0,1%SDS/0,25 lait écrémé pendant 2h à 65°C. La sonde EPSPS d'*Arabidopsis thaliana* a été marquée au ³²P-dCTP par "random-priming" selon les instructions du fournisseur (Kit Ready to Go de Pharmacia). L'activité spécifique obtenue est de l'ordre de 10⁸ cpm par µg de fragment. Après dénaturation pendant 5 min à 100°C, la sonde est ajoutée dans le milieu de préhybridation et l'hybridation est poursuivie pendant 14 heures à 55°C. Les filtres sont fluorographiés 48h à -80°C avec un film KodakXAR5 et des écrans renforçateurs Hyperscreen RPN d'Amersham. L'alignement des spots positifs sur le filtre avec les boîtes d'où ils sont issus permet de prélever, sur la boîte, des zones correspondant aux phages présentant une réponse d'hybridation positive avec la sonde EPSPS d'*Arabidopsis thaliana*. Cette étape d'étalement, transfert, hybridation, récupération est répétée jusqu'à ce que tous les spots de la boîte des phages successivement purifiés se révèlent positifs à 100% en hybridation. Une plage de lyse par phage indépendant est alors prélevée dans du milieu λ diluant (Tris-Cl pH= 7,5; MgSO4 10mM; NaCl 0,1M; gélatine 0,1%), ces phages en solution constituant les clones positifs de l'EPSPS de la suspension cellulaire de maïs.

### 4. Préparation et analyse de l'ADN des clones EPSPS de la suspension cellulaire de maïs BMS.

On ajoute environ 5.10⁸ phages à 20 ml de bactéries C600hfl à 2 OD 600nm/ml et incubés 15 minutes à 37°C. Cette suspension est alors diluée dans 200ml de milieu de croissance des bactéries dans un Erlen de 11 et agitée dans un agitateur rotatif à 250 rpm. La lyse est constatée par clarification du milieu, correspondant à 1 lyse des bactéries turbides et se produit après environ 4 h d'agitation. Ce surnageant est alors traité comme décrit dans "Current Protocols in Molecular Biology". L'ADN obtenu correspond aux clones d'EPSPS de la suspension cellulaire de maïs BMS.

Un à deux µg de cet ADN sont coupés par EcoRI et séparés sur gel d'agarose LGTA/TBE (réf. CPMB) à 0,8%. Une dernière vérification consiste à s'assurer que l'ADN purifié présente bien un signal d'hybridation avec la sonde EPSPS d'*Arabidopsis thaliana*. Après l'électrophorèse, les fragments d'ADN sont transférés sur membrane Hybond N d'Amersham selon le protocole de Southern décrit dans "Current Protocols in Molecular Biology". Le filtre est hybridé avec la sonde EPSPS d'*Arabidopsis thaliana* selon les conditions décrites au paragraphe 3 ci-dessus. Le clone présentant un signal d'hybridation avec la sonde EPSPS d'*Arabidopsis thaliana* et contenant le plus long fragment EcoRI a une taille estimée sur gel à environ 1,7kpb.

### 5. Obtention du clone pRPA-ML-711:

Dix µg de l'ADN du clone phagique contenant l'insert de 1,7kpb sont digérés par EcoRI et séparés sur gel d'agarose LGTA/TBE (réf. CPMB) à 0,8%. Le fragment de gel contenant l'insert de 1,7kpb est excisé du gel par coloration BET et le fragment est traité à la β-agarse selon le protocole du fournisseur New England Biolabs. L'ADN purifié du fragment de 1,7kpb est ligué à 12°C pendant 14h avec l'ADN du plasmide pUC 19 (New England Biolabs) coupé par EcoRI selon le protocole de ligation décrit dans "Current Protocols in Molecular Biology". Deux µl du mélange de ligation ci-dessus sont utilisés pour la transformation d'une aliquote d'E.coli DH10B électro compétentes ; la transformation se fait par électroporation en utilisant les conditions suivantes: le mélange de bactéries compétentes et de milieu de ligation est introduit dans une cuvette d'électroporation d'épaisseur 0,2cm (Biorad) préalablement refroidie à 0°C. Les conditions physiques de l'électroporation utilisant un électroporateur de marque Biorad sont 2500 Volts, 25 µFarad et 200 Ω. Dans ces conditions, le temps de décharge moyen de condensateur est de l'ordre de 4,2 millisecondes. Les bactéries sont alors reprises dans 1 ml de milieu SOC (réf. CPMB) et agitées pendant 1 heure à 200 rpm sur un agitateur rotatif dans des tubes Corning de 15 ml. Après étalement sur milieu LB/agar supplémenté à 100 µg/ml de carbéniciline, les mini-préparations des clones bactériens ayant poussé après une nuit à 37 °C est réalisée selon le protocole décrit dans "Current Protocols in Molecular Biology". Après digestion par EcoRI de l'ADN et séparation en électrophorèse sur gel d'agarose LGTA/TBE (réf. CPMB) à 0,8%, les clones présentant un insert de 1,7kpb sont conservés. Une dernière vérification consiste à s'assurer que l' ADN purifié présente bien un signal d'hybridation avec la sonde EPSPS d'*Arabidopsis thaliana*. Après l'électrophorèse, les fragments d'ADN sont transférés sur membrane Hybond N d'Amersham selon le protocole de Southern décrit dans "Current Protocols in Molecular Biology". Le filtre est hybridé avec la sonde EPSPS d'*Arabidopsis thaliana* selon les conditions décrites au paragraphe 3 ci-dessus. Le clone plasmidique présentant un insert de 1,7kpb et hybridant avec la sonde EPSPS d'*Arabidopsis thaliana a* été préparé à plus grande échelle et l'ADN résultant de la lyse des bactéries purifié sur gradient de CsCl ainsi que décrit dans "Current Protocols in Molecular Biology". L'ADN purifié a été partiellement séquencé avec un kit Pharmacia en suivant les instructions du fournisseur et en utilisant comme amorces, les amorces universelles de M13 directes et inverses commandées chez le même fournisseur. La séquence partielle réalisée couvre environ 0,5 kpb. La séquence dérivée en acides aminés dans la région de la protéine mature (environ 50 résidus acides aminés) présente une identité de 100% avec la séquence aminée correspondante de l'EPSPS mature de maïs décrite dans le brevet américain USP 4 971 908). Ce clone correspondant à un fragment EcoRI de 1,7kpb de l'ADN de 1' EPSP de la suspension cellulaire de maïs BMS a été nommé **pRPA-ML-711.** La séquence complète de ce clone a été réalisée sur les deux brins en utilisant le protocole du kit Pharmacia et en synthétisant des oligonucléotides complémentaires et de direction opposée tous les 250 pb environ. La séquence complète de ce clone de 1713 pb obtenue est présentée par SEQ ID N° 1.

### 6. Obtention du clone pRPA-ML-715:

L'analyse de la séquence du clone pRPA-ML-711 et en particulier la comparaison de la séquence d' acides aminés dérivés avec celle de maïs montre une extension de séquence de 92 pb en amont du codon GCG codant pour l'Alanine NH2-terminale de la partie mature de l'EPSPS de maïs (brevet américain USP 4 971 908). De même une extension de 288 pb en aval du codon AAT codant pour l'asparagine COOH-terminale de la partie mature de l'EPSPS de maïs (brevet américain USP 4 971 908) est observée. Ces deux parties pourraient correspondre, pour l'extension NH2-terminale à une portion de la séquence d'un peptide de transit pour la localisation plastidiale et pour l'extension COOH-terminale à la région 3' non traduite de l'ADNc.

Afin d'obtenir un ADNc codant pour la partie mature de l'ADNc de l'EPSPS de maïs, telle que décrite dans l'USP 4 971 908, les opérations suivantes ont été réalisées:

### a) Elimination de la région 3' non traduite: construction de pRPA-ML-712:

Le clone pRPA-ML-711 a été coupé par l'enzyme de restriction AseI et les extrémités résultant de cette coupure rendues franches par traitement avec le fragment de Klenow de l'ADN polymérase I selon le protocole décrit dans CPMB. Une coupure par l'enzyme de restriction SacII a ensuite été effectuée. L'ADN résultant de ces opérations a été séparé par électrophorèse sur gel d'agarose LGTA/TBE (réf. CPMB) 1%.

Le fragment de gel contenant l'insert "AseI-extrémités franches/SacII" de 0,4 kpb a été excisé du gel et purifié selon le protocole décrit au paragraphe 5 ci-dessus. L'ADN du clone pRPA-ML-711 a été coupé par l'enzyme de restriction HindIII située dans le polylinker du vecteur de clonage pUC19 et les extrémités résultant de cette coupure ont été rendues franches par traitement avec le fragment de Klenow de l'ADN polymérase I. Une coupure par l'enzyme de restriction SacII a ensuite été effectuée. L'ADN résultant de ces manipulations a été séparé par électrophorèse sur gel d'agarose LGTA/TBE (réf. CPMB) 0,7%.

Le fragment de gel contenant l'insert HindIII-extrémités franches/SacII de environ 3,7kpb a été excisé du gel et purifié selon le protocole décrit au paragraphe 5 ci-dessus.

Les deux inserts ont été ligués, et 2 µl du mélange de ligation ont servi à transformer E. *coli* DHlOB ainsi que décrit plus haut au paragraphe 5.

On analyse le contenu en ADN plasmidique de différents clones selon la procédure décrite pour pRPA-ML-711. Un des clones plasmidiques retenu contient un insert EcoRI-HindIII de 1,45 kpb environ. La séquence des extrémités terminales de ce clone révèle que l'extrémité 5' de l'insert correspond exactement à l'extrémité correspondante de pRPA-ML-711 et que l'extrémité 3' terminale présente la séquence suivante:

" 5'-...AATTAAGCTCTAGAGTCGACCTGCAGGCATGCAAGCTT-3' ".

La séquence soulignée correspond au codon de l'acide aminé COOH-terminal asparagine, le codon suivant correspondant au codon stop de la traduction. Les nucléotides en aval correspondent à des éléments de séquence du polylinker de pUCI9. Ce clone comprenant la séquence de pRPAML-711 jusqu'au site de terminaison de la traduction de l'EPSPS mature de maïs et suivie de séquences du polylinker de pUC 19 jusqu'au site HindIII a été nommé **pRPA-ML-712.**

### b) Modification de l'extrémité 5' de pRPA-ML-712: construction de pRPA-ML-715

Le clone pRPA-ML-712 a été coupé par les enzymes de restrictions PstI et HindIII. L'ADN résultant de ces manipulations a été séparé par électrophorèse sur gel d'agarose LGTA/TBE (réf. CPMB) 0,8%. Le fragment de gel contenant l'insert PstI/EcoRI de 1,3 kpb a été excisé du gel et purifié selon le protocole décrit au paragraphe 5 ci-dessus. Cet insert a été mis en ligation en présence de quantité équimoléculaire de chacun des deux oligonucléotides partiellement complémentaires, de séquence: Oligo1: 5'-GAGCCGAGCTCCATGGCCGGCGCCGAGGAGATCGTGCTGCA-3' Oligo 2: 5'-GCACGATCTCCTCGGCGCCGGCCATGGAGCTCGGCTC-3' ainsi qu'en présence d'ADN du plasmide pUC19 digéré par les enzymes de restrictions BamHI et HindIII.

Deux µl du mélange de ligation ont servi à transformer *E. coli* DHlOB ainsi que décrit plus haut au paragraphe 5. Après analyse du contenu en ADN plasmidique de différents clones selon la procédure décrite ci-dessus au paragraphe 5, un des clones présentant un insert d'environ 1,3 kpb a été conservé pour analyses ultérieures. La séquence de l'extrémité 5' terminale du clone retenu révèle que la séquence ADN dans cette région est la suivante: séquence du polylinker de pUC19 des sites EcoRI à BamHI, suivi de la séquence des oligonucléotides utilisés lors du clonage, suivi du reste de la séquence présente dans pRPAML-712. **Ce clone a été nommé pRPA-ML-713.** Ce clone présente un codon methionine ATG inclus dans un site NcoI en amont du codon Alanine N-terminal de l'EPSPSynthase mature. De plus, les codons alanine et glycine de l'extrémité N-terminale ont été conservées, mais modifiées sur la troisième base variable : GCGGGT initial donne GCCGGC modifié.

**Le clone pRPA-ML-713** a été coupé par l'enzyme de restriction HindIII et les extrémités de cette coupure rendues franches par traitement avec le fragment de Klenow de la ADN polymérase I. Une coupure par l'enzyme de restriction SacI a ensuite été effectuée. L'ADN résultant de ces manipulations a été séparé par électrophorèse sur gel d'agarose LGTA/TBE (réf. CPMB) 0,8%. Le fragment de gel contenant l'insert "HindIII-extrémités franches/SacI" de 1,3 kpb a été excisé du gel et purifié selon le protocole décrit au paragraphe 5 ci-dessus. Cet insert a été mis en ligation en présence d'ADN du plasmide pUC19 digéré par l'enzyme de restriction XbaI et les extrémités de cette coupure rendues franches par traitement avec le fragment de Klenow de l'ADN polymérase I. Une coupure par l'enzyme de restriction SacI a ensuite été effectuée. Deux µl du mélange de ligation ont servi à transformer *E. coli* DHlOB ainsi que décrit plus haut au paragraphe 5. Après analyse du contenu en ADN plasmidique de différents clones selon la procédure décrite ci-dessus au paragraphe 5, un des clones présentant un insert d'environ 1,3 kpb a été conservé pour analyses ultérieures. La séquence des extrémités terminales du clone retenu révèle que la séquence ADN est la suivante: séquence du polylinker de pUC19 des sites EcoRI à SacI, suivie de la séquence des oligonucléotides utilisés lors du clonage délétée des 4 pb GATCC de l'oligonucléotide **1** décrit ci-dessus, suivi du reste de la séquence présente dans pRPA-ML-712 jusqu'au site HindIII et séquence du polylinker de pUC19 de Xbal à HindIII. **Ce clone a été nommé pRPA-ML-715.**

### 7) Obtention d'un ADNc codant pour une EPSPS de maïs mutée

Toutes les étapes de mutagenèse ont été réalisées avec le U.S.E. mutagenesis kit de Pharmacia en suivant les instructions du fournisseur. Le principe de ce système de mutagenèse est le suivant: l'ADN plasmidique est dénaturé par la chaleur et réassocié en présence d'un excès molaire d'une part de l'oligonucléotide de mutagenèse, et d'autre part d'un oligonucléotide permettant d'éliminer un site d'enzyme de restriction unique présent dans le polylinker. Après l'étape de réassociation, la synthèse du brin complémentaire est réalisée par l'action de la T4 ADN polymérase en présence de T4 ADN ligase et de protéine du gène 32 dans un tampon approprié fourni. Le produit de synthèse est incubé en présence de l'enzyme de restriction, dont le site est supposé avoir disparu par mutagenèse. La souche d'*E*. *coli* présentant, en particulier, la mutation mutS est utilisée comme hôte pour la transformation de cet ADN. Après croissance en milieu liquide, l'ADN plasmidique total est préparé, incubé en présence de l'enzyme de restriction utilisée précédemment. Après ces traitements, la souche d'*E*. *coli* DHlOB est utilisée comme hôte pour la transformation. L'ADN plasmidique des clones isolés est préparé et la présence de la mutation introduite vérifiée par séquençage.

### A)- modifications de sites ou de séquence sans incidence a priori sur le caractère de résistance de l'EPSPS de maïs aux produits inhibiteurs compétitifs de l'activité EPSP synthase: élimination d'un site NcoI interne de pRPA-ML-715.

La séquence de pRPA-ML-715 est numérotée arbitrairement en plaçant la première base du codon Alanine N-terminal GCC en position 1. Cette séquence présente un site NcoI en position 1217. L'oligonucléotide de modification du site présente la séquence :

5'-CCACAGGATGGCGATGGCCTTCTCC-3'.

Après séquençage selon les références données ci-dessus, la séquence lue après mutagenèse correspond à celle de l'oligonucléotide utilisé. Le site NcoI a bien été éliminé et la traduction en acides aminés dans cette région conserve la séquence initiale présente sur pRPA-ML-715.

Ce clone a été nommé pRPA-ML-716.

La séquence de 1340 bp de ce clone est présentée SEQ ID N° 2 et SEQ ID N° 3.

### B) modifications de séquence permettant l'augmentation du caractère de résistance de l'EPSPS de maïs aux produits inhibiteurs compétitifs de l'activité EPSP synthase.

Les oligonucléotides suivants ont été utilisés :
a) mutation Thr 102 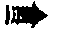 Ile. 5'-GAATGCTGGAATCGCAATGCGGCCATTGACAGC-3'
b) mutation Pro 106 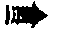 Ser. 5'-GAATGCTGGAACTGCAATGCGGTCCTTGACAGC-3'
c) mutations Gly 101 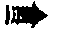 Ala et Thr 102 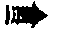 Ile. 5'-CTTGGGGAATGCTGCCATCGCAATGCGGCCATTG-3'
d) mutations Thr 102 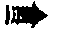 Ile et Pro 106 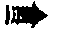 Ser.

5'-GGGGAATGCTGGAATCGCAATGCGGTCCTTGACAGC-3'

Après séquençage, la séquence lue après mutagenèse sur les trois fragments mutés est identique à la séquence de l'ADN parental pRPA-ML-716 à l'exception de la région mutagenéisée qui correspond à celle des oligonucléotides de mutagenèse utilisés. Ces clones ont été nommés : pRPA-ML-717 pour la mutation Thr 102 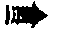 Ile, pRPA-ML-718 pour la mutation Pro 106 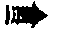 Ser, pRPA-ML-719 pour les mutations Gly 101 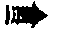 Ala et Thr 102 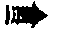 Ile et pRPA-ML-720 pour les mutations Thr 102 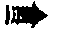 Ile et Pro 106 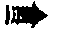 Ser.

La séquence de 1340 bp de pRPA-ML-720 est présentée SEQ ID N° 4 et SEQ ID N° 5.

L'insert NcoI-HindIII de 1395 pb sera nommé dans la suite des descriptions "le double mutant de l'EPSPS de maïs".

### EXEMPLE 2: Construction de gènes chimères

On effectue la construction de gènes chimères selon l'invention à partir des éléments suivants:
1) On isole le clone génomique (clone cosmide c22) d'*Arabidopsis thaliana* contenant deux gènes du type "H3.3-like" comme décrit dans Chaubet et al (J. Mol. Biol. 1992. **225** 569-574).
**2) intron n°1:**
   On purifie un fragment d'ADN de 418 paires de bases à partir de la digestion du clone cosmide c22 avec l'enzyme de restriction *DdeI* suivie d'un traitement avec un fragment de Klenow d'ADN polymérase de *E.coli*., selon les instructions du fabricant pour créer un fragment d'ADN à extrémité franche puis coupé avec *MseI.* Le fragment ADN purifié est ligaturé à un adaptateur oligonucléotide synthétique ayant la séquence suivante: 
adaptateur 1: 5' TAATTTGTTGAACAGATCCC 3'
TAAACAACTTGTCTAGGG
   Le produit de ligation est cloné dans pGEM7Zf(+) (catalogue Stratagene n° P2251) qui a été digéré par *SmaI*. Ce clone, appelé "intron n°1", est vérifié par séquençage (SEQ ID 6).
**3) intron n°2:**
   On purifie un fragment d'ADN de 494 paires de bases à partir de la digestion du clone cosmide c22 avec les enzymes de restriction *AluI* et *CfoI*. Le fragment ADN purifié est ligaturé à un adaptateur oligonucléotide synthétique ayant la séquence suivante: 
adaptateur 2: 5' CAGATCCCGGGATCTGCG 3'
GCGTCTAGGGCCCTAGACGC
   Le produit de ligation est cloné dans pGEM7Zf(+) (catalogue Stratagene n° P2251) qui a été digéré par *SmaI*. Ce clone, appelé "intron n°2", est vérifié par séquençage (SEQ ID 7).
**4) pRA-1**
   La construction de ce plasmide est décrite dans la demande française 9308029. Ce plasmide est un dérivé de pBI 101.1 (Catalogue Clonetech n° 6017-1) qui contient le promoteur d'histone d'*Arabidopsis* H4A748 régulant la synthèse du gène de la β-glucoronidase de *E.coli* et du site de polyadénylation de la nopaline synthase (NOS"). Ainsi on obtient un gène chimère de structure:
   " promoteur H4A748- gène GUS- NOS"
**5). pCG-1**
   Ce plasmide contient l'intron n°1 ci-dessus placé entre le promoteur H4A748 et la région codante GUS de pRA-1. Ce plasmide est obtenu par digestion de clone cosmide c22 avec *BamHI* et *SmaI*. L'intron n°1 de 418 paires de bases est directement ligaturé dans pRA-1 qui a été digéré avec *BamHI* et *Smal*.
   Ainsi on obtient un gène chimère de structure:
   " promoteur H4A748- intron n°1-gène GUS- NOS"
**6). pCG-13**
   Ce plasmide contient l'intron n°2 ci-dessus placé entre le promoteur H4A748 et la région codante GUS de pRA-1. Ce plasmide est obtenu par digestion de clone cosmide c22 avec *BamHI* et *SmaI*. L'intron n°2 de 494 paires de bases est directement ligaturé dans pRA-1 qui a été digéré avec *BamHI* et *SmaI*.
   Ainsi on obtient un gène chimère de structure:
   " promoteur H4A748- intron n°2-gène GUS- NOS"
**7). pCG-15**
   Ce plasmide contient seulement l'intron n°1 devant la séquence codante GUS de ci-dessus placé entre le promoteur H4A748 et la région codante GUS de pCG-1. Ce plasmide est obtenu par digestion de pCG-1 avec *BamHI* et *HindIII* suivie d'un traitement avec un fragment de Klenow d'ADN polymérase de *E.coli*., selon les instructions du fabricant pour créer un fragment d'ADN à extrémité franche.
   Ce vecteur est alors religaturé pour donner un gène chimère de structure:
   "intron n°1-GUS-NOS"
**8). pCG-18**
   Ce plasmide contient seulement l'intron n°2 ci-dessus devant la séquence codante GUS de pCG-13. Ce plasmide est obtenu par digestion partielle de pCG-13 avec *BamHI* et *Sphl.* suivie d'un traitement avec un fragment de d'ADN polymérase de phage T4, selon les instructions du fabricant pour créer un fragment d'ADN à extrémité franche.
   Ce vecteur est alors religaturé et vérifié par digestion enzymatique pour donner un gène chimère de structure:
   "intron n°2-GUS-NOS"
**9). pRPA-RD-124**
   Addition d'un signal de polyadénylation "nos" à pRPA-ML-720 avec création d'une cassette de clonage contenant le gène d'EPSPS double mutant de maïs (Thr 102 → Ile et Pro 106 → Ser). pRPA-ML-720 est digéré avec Hind III, traité avec le fragment de Klenow de l'ADN polymérase I d'*E*. *coli* pour produire une extrémité franche. On effectue une seconde digestion avec Nco I et le fragment EPSPS est purifié. Le gène EPSPS est ensuite ligué avec pRPA-RD-12 purifié (une cassette de clonage contenant le signal de polyadénylation de la nopaline synthase) pour donner pRPA-RD-124. Pour obtenir le vecteur pRPA-RD-12 purifié utile, il a fallu que celui-ci soit préalablement digéré par SalI, traité avec l'ADN polymérase de Klenow, puis digéré une seconde fois avec NcoI.
**10). pRPA-RD-125**
   Addition d'un peptide de transit optimisé (PTO) à pRPA-RD-124 avec création d'une cassette de clonage contenant le gène d'EPSPS ciblé sur les plasmides. pRPA-RD-7 (demande de brevet européen EP 652 286) est digérée avec Sph I, traité avec la T4 ADN polymérase, puis digérée avec Spe 1 et le fragment PTO est purifié. Ce fragment PTO est cloné dans pRPA-RD-124 qui a été préalablement digérée par NcoI, traité avec l'ADN polymérase de Klenow pour enlever la partie protubérante 3', puis digérée par Spe I. Ce clone est alors séquencé pour assurer la fusion traductionnelle correcte entre le PTO et le gène d'EPSPS. On obtient alors pRPA-RD-125.
**11). pRPA-RD-196**
   Dans ce plasmide, la partie "intron n°1 + gène de la β-glucoronidase de *E.coli*." de pCG-1 est remplacée par un gène chimère de 2 kilobases contenant un peptide de transit optimisé de gène d'EPSPS double mutant (Ile₁₀₂ + Ser₁₀₆) et un site de polyadénylation de la nopaline synthase ("NOS") isolé de pRPA-RD-125. Pour obtenir pRPA-RD-196, on effectue la digestion de pCG-1 avec *EcoRI* et *BamHI*, suivie d'un traitement avec un fragment de klenow d'ADN polymérase de *E.coli*., selon les instructions du fabricant pour créer un fragment d'ADN à extrémité franche. Le fragment d'ADN de 2 kilobases contenant un peptide de transit optimisé de gène d'EPSPS double mutant (Ile₁₀₂ + Ser₁₀₆ et un site de polyadénylation de la nopaline synthase ("NOS") est obtenu à partir de pRPA-RD-125 par digestion avec *NcoI* et *NotI*, suivie d'un traitement avec l'ADN polymérase de *E.coli*., selon les instructions du fabricant pour créer un fragment d'ADN à extrémité franche. Ce fragment à extrémité franche est alors ligaturé dans pCG-1 préparé ci-dessus.
   Ainsi on obtient un gène chimère de structure:
   " promoteur H4A748 - PTO - gène EPSPS de maïs - NOS"
**12). pRPA-RD-197**
   Dans ce plasmide, la partie "gène de la β-glucoronidase de *E.coli*." de pCG-1 est remplacée par un gène chimère de 2 kilobases contenant un peptide de transit optimisé, un gène d'EPSPS double mutant (Ile₁₀₂ + Ser₁₀₆) et un site de polyadénylation de la nopaline synthase ("NOS") isolé de pRPA-RD-125. Pour obtenir pRPA-RD-197, on effectue la digestion de pCG-1 avec *EcoRI* suivie d'un traitement avec un fragment de Klenow d'ADN polymérase de *E.coli*., selon les instructions du fabricant pour créer un fragment d'ADN à extrémité franche puis coupé avec *SmaI*. Le fragment d'ADN de 2 kilobases contenant un peptide de transit optimisé, un gène d'EPSPS double mutant (Ile₁₀₂ + Ser₁₀₆) et un site de polyadénylation de la nopaline synthase ("NOS") est obtenu à partir de pRPA-RD-125 par digestion avec *NcoI* et *NotI*, suivie d'un traitement avec l'ADN polymérase de *E. coli*., selon les instructions du fabricant pour créer un fragment d'ADN à extrémité franche. Ce fragment à extrémité franche est alors ligaturé dans pCG-1 préparé ci-dessus.
   Ainsi on obtient un gène chimère de structure:
   " promoteur H4A748 - intron n°1 - gène EPSPS de maïs - NOS"
**13). pRPA-RD-198**
   Dans ce plasmide, la partie "gène de la β-glucoronidase de *E.coli*." de pCG-13 est remplacée par un gène chimère de 2 kilobases contenant un peptide de transit optimisé, un gène d'EPSPS double mutant (Ile₁₀₂ + Ser₁₀₆) et un site de polyadénylation de la nopaline synthase ("NOS") isolé de pRPA-RD-125. Pour obtenir pRPA-RD-198, on effectue la digestion de pCG-13 avec *EcoRI* suivie d'un traitement avec un fragment de Klenow d'ADN polymérase de *E.coli*., selon les instructions du fabricant pour créer un fragment d'ADN à extrémité franche puis coupé avec *SmaI*. Le fragment d'ADN de 2 kilobases contenant un peptide de transit optimisé, un gène d'EPSPS double mutant (Ile₁₀₂ + Ser₁₀₆) et un site de polyadénylation de la nopaline synthase ("NOS") est obtenu à partir de pRPA-RD-125 par digestion avec *NcoI* et *NotI*, suivie d'un traitement avec l'ADN polymérase de *E.coli*., selon les instructions du fabricant pour créer un fragment d'ADN à extrémité franche. Ce fragment à extrémité franche est alors ligaturé dans pCG-13 préparé ci-dessus.
   Ainsi on obtient un gène chimère de structure:
   " promoteur H4A748 - intron n°2- PTO- gène EPSPS de maïs - NOS"

### EXEMPLE 3: Expression de l'activité d'un gène rapporteur

### 1) Transformation et régénération

Le vecteur est introduit dans la souche non oncogène d'*Agrobacterium tumefaciens* LBA 4404 disponible sur catalogue (Clontech #6027-1) par croisement triparental à l'aide du plasmide "helper" pRK 2013 dans *Escherichia coli* HB101 selon la procédure décrite par Bevan M. (1984) Nucl.Acids Res., 12, 8711-8721.

La technique de transformation à partir d'explants racinaires d'*Arabidopsis thaliana* L.-écotype C24 a été effectuée selon la procédure décrite par Valvekens D. et al. (1988) Proc.Natl.Acad.Sci USA, 85, 5536-5540. Brièvement, 3 étapes sont nécessaires: induction de la formation des cals sur milieu B5 de Gamborg complémenté de 2,4-D et de kinétine; formation de bourgeons sur milieu B5 de Gamborg complémenté de 2iP et d'IAA; enracinement et formation de graines sur MS sans hormones.

### 2) Mesure de l'activité GUS dans les plantes

### a- observations histochimiques

La révélation de l'activité GUS par tache histochimique (Jefferson R.A. et al. (1987) EMBO J., **6**, 3901-3907) sur des plantes transgéniques de 10 jours montre une augmentation de l'intensité du patron histochimique spécifique de tissu pour les plasmides contenant les séquences d'intron (pCG-1 et pCG-13) par rapport à ceux sans ces introns (pRA-1). En particulier le patron de tache pour pCG-1 et pCG-13 est identique, montrant une augmentation d'intensité des taches des tissus vasculaires et méristématiques, feuilles et racines par rapport à celui de la construction pRA-1. Les constructions contenant seulement les séquences d'intron n°1 (pCG-15 et pCG-18 montrent une tache histochimique extrêmement claire seulement dans la région du méristème apical.

### b- mesures fluorométriques

L'activité GUS mesurée par fluorométrie sur des extraits de bourgeons floraux et de feuilles de la rosette (Jefferson R.A. et al. (1987) EMBO J., **6**, 3901-3907) à partir de 12 plantes, montre que l'activité du promoteur H4A748 est augmentée sous l'influence des introns n° 1 et 2. Par rapport à la construction pRA-1, l'activité GUS de pCG-1 et pCG-13 sont au moins six fois supérieures dans les bourgeons floraux, vingt fois supérieures dans les feuilles de la rosette et vingt six fois supérieures dans les racines.

Ces mesures montrent clairement que les introns n°1 et n°2 de gènes d'histone d'arabidopsis de type "es" utilisé comme zone de régulation induisent une augmentation de l'activité de l'expression du gène chimère.

### EXEMPLE 4: Tolérance de plantes transgéniques à un herbicide

### 1) Transformation et régénération

Le vecteur est introduit dans la souche non oncogène d'*Agrobacterium tumefaciens* LBA 4404 disponible sur catalogue (Clontech #6027-1) par croisement triparental à l'aide du plasmide "helper" pRK 2013 dans *Escherichia coli* HB101 selon la procédure décrite par Bevan M. (1984) Nucl.Acids Res., **12,** 8711-8721.

La technique de transformation à partir d'explants foliaires de tabac est basée sur la procédure décrite par Horsh R. et al. (1985) Science, **227**, 1229-1231. La régénération du tabac PBD6 (provenance SEITA-France) à partir d'explants foliaires est réalisée sur un milieu de base Murashige et Skoog (MS) comprenant 30g/l de saccharose ainsi que 200µg/ml de kanamycine en trois étapes successives: la première comprend l'induction des pousses sur un milieu MS additionné de 30g de saccharose contenant 0,05mg d'acide naphtylacétique (ANA) et 2mg/l de benzylaminopurine (BAP) pendant 15 jours. Les pousses formées au cours de cette étape sont ensuite développées par culture sur un milieu MS additionné de 30g/l de saccharose mais ne contenant pas d'hormone, pendant 10 jours. Puis on prélève des pousses développées et on les cultive sur un milieu d'enracinement MS dilué au demi, à teneur moitié en sels, vitamines et sucres et ne contenant pas d'hormone. Au bout d'environ 15 jours, les pousses enracinées sont passées en terre.

### 2) Mesure de la tolérance au glyphosate:

Vingt plantes transformées ont été régénérées et passées en serre pour chacune des constructions pRPA-RD-196, pRPA-RD-197 et pRPA-RD-198. Ces plantes ont été traitées en serre au stade 5 feuilles avec une suspension aqueuse de l'herbicide, vendu sous la marque RoundUp, correspondant à 0,8kg de matière active glyphosate par hectare.

Les résultats correspondent à l'observation d'indices de phytotoxicité relevés 3 semaine après traitement. Dans ces conditions, on constate que les plantes transformées par les constructions présentent en moyenne une tolérance acceptable (pRPA-RD-196) voire bonne (pRPA-RD-197 et pRPA-RD-198) alors que les plantes témoins non transformées sont complètement détruites.

Ces résultats montrent clairement l'amélioration apportée par l'utilisation d'un gène chimère selon l'invention pour un même gène codant pour la tolérance au glyphosate.

Les plantes transformées selon l'invention peuvent être utilisées comme parents pour l'obtention de lignées et d'hybrides ayant le caractère phénotypique correspondant à l'expression du gène chimère introduit.

### Liste des séquences:

SEQ ID N° 1.

AATCAATTTC ACACAGGAAA CAGCTATGAC CATGATTACG AATTCGGGCC CGGGCGCGTG 60
ATCCGGCGGC GGCAGCGGCG GCGGCGGTGC AGGCGGGTGC CGAGGAGATC GTGCTGCAGC 120
CCATCAAGGA GATCTCCGGC ACCGTCAAGC TGCCGGGGTC CAAGTCGCTT TCCAACCGGA 180
TCCTCCTACT CGCCGCCCTG TCCGAGGGGA CAACAGTGGT TGATAACCTG CTGAACAGTG 240
AGGATGTCCA CTACATGCTC GGGGCCTTGA GGACTCTTGG TCTCTCTGTC GAAGCGGACA 300
AAGCTGCCAA AAGAGCTGTA GTTGTTGGCT GTGGTGGAAA GTTCCCAGTT GAGGATGCTA 360
AAGAGGAAGT GCAGCTCTTC TTGGGGAATG CTGGAACTGC AATGCGGCCA TTGACAGCAG 420
CTGTTACTGC TGCTGGTGGA AATGCAACTT ACGTGCTTGA TGGAGTACCA AGAATGAGGG 480
AGAGACCCAT TGGCGACTTG GTTGTCGGAT TGAAGCAGCT TGGTGCAGAT GTTGATTGTT 540
TCCTTGGCAC TGACTGCCCA CCTGTTCGTG TCAATGGAAT CGGAGGGCTA CCTGGTGGCA 600
AGGTCAAGCT GTCTGGCTCC ATCAGCAGTC AGTACTTGAG TGCCTTGCTG ATGGCTGCTC 660
CTTTGGCTCT TGGGGATGTG GAGATTGAAA TCATTGATAA ATTAATCTCC ATTCCGTACG 720
TCGAAATGAC ATTGAGATTG ATGGAGCGTT TTGGTGTGAA AGCAGAGCAT TCTGATAGCT 780
GGGACAGATT CTACATTAAG GGAGGTCAAA AATACAAGTC CCCTAAAAAT GCCTATGTTG 840
AAGGTGATGC CTCAAGCGCA AGCTATTTCT TGGCTGGTGC TGCAATTACT GGAGGGACTG 900
TGACTGTGGA AGGTTGTGGC ACCACCAGTT TGCAGGGTGA TGTGAAGTTT GCTGAGGTAC 960
TGGAGATGAT GGGAGCGAAG GTTACATGGA CCGAGACTAG CGTAACTGTT ACTGGCCCAC 1020
CGCGGGAGCC ATTTGGGAGG AAACACCTCA AGGCGATTGA TGTCAACATG AACAAGATGC 1080
CTGATGTCGC CATGACTCTT GCTGTGGTTG CCCTCTTTGC CGATGGCCCG ACAGCCATCA 1140
GAGACGTGGC TTCCTGGAGA GTAAAGGAGA CCGAGAGGAT GGTTGCGATC CGGACGGAGC 1200
TAACCAAGCT GGGAGCATCT GTTGAGGAAG GGCCGGACTA CTGCATCATC ACGCCGCCGG 1260
AGAAGCTGAA CGTGACGGCG ATCGACACGT ACGACGACCA CAGGATGGCC ATGGCCTTCT 1320
CCCTTGCCGC CTGTGCCGAG GTCCCCGTCA CCATCCGGGA CCCTGGGTGC ACCCGGAAGA 1380
CCTTCCCCGA CTACTTCGAT GTGCTGAGCA CTTTCGTCAA GAATTAATAA AGCGTGCGAT 1440
ACTACCACGC AGCTTGATTG AAGTGATAGG CTTGTGCTGA GGAAATACAT TTCTTTTGTT 1500
CTGTTTTTCT CTTTCACGGG ATTAAGTTTT GAGTCTGTAA CGTTAGTTGT TTGTAGCAAG 1560
TTTCTATTTC GGATCTTAAG TTTGTGCACT GTAAGCCAAA TTTCATTTCA AGAGTGGTTC 1620
GTTGGAATAA TAAGAATAAT AAATTACGTT TCAGTGAAAA AAAAAAAAAA AAAAAAAAAA 1680
AAAAAAAAAA AAAAAAAAAA AACCCGGGAA TTC 1713

SEQ ID N° 2.

CCATG GCC GGC GCC GAG GAG ATC GTG CTG CAG CCC ATC AAG GAG ATC 47
Ala Gly Ala Glu Glu Ile Val Leu Gln Pro Ile Lys Glu Ile
1               5                   10

TCC GGC ACC GTC AAG CTG CCG GGG TCC AAG TCG CTT TCC AAC CGG ATC 95
Ser Gly Thr Val Lys Léu Pro Gly Ser Lys Ser Leu Ser Asn Arg Ile
15 20 25 30

CTC CTA CTC GCC GCC CTG TCC GAG GGG ACA ACA GTG GTT GAT AAC CTG 143
Leu Leu Leu Ala Ala Leu Ser Glu Gly Thr Thr Val Val Asp Asn Leu
35 40 45

CTG AAC AGT GAG GAT GTC CAC TAC ATG CTC GGG GCC TTG AGG ACT CTT 191
Leu Asn Ser Glu Asp Val His Tyr Met Leu Gly Ala Leu Arg Thr Leu
50 55 60

GGT CTC TCT GTC GAA GCG GAC AAA GCT GCC AAA AGA GCT GTA GTT GTT 239
Gly Leu Ser Val Glu Ala Asp Lys Ala Ala Lys Arg Ala Val Val Val
65 70 75

GGC TGT GGT GGA AAG TTC CCA GTT GAG GAT GCT AAA GAG GAA GTG CAG 287
Gly Cys Gly Gly Lys Phe Pro Val Glu Asp Ala Lys Glu Glu Val Gln
80 85 90

CTC TTC TTG GGG AAT GCT GGA ACT GCA ATG CGG CCA TTG ACA GCA GCT 335
Leu Phe Leu Gly Asn Ala Gly Thr Ala Met Arg Pro Leu Thr Ala Ala
95 100 105 110

GTT ACT GCT GCT GGT GGA AAT GCA ACT TAC GTG CTT GAT GGA GTA CCA 383
Val Thr Ala Ala Gly Gly Asn Ala Thr Tyr Val Leu Asp Gly Val Pro
115 120 125

AGA ATG AGG GAG AGA CCC ATT GGC GAC TTG GTT GTC GGA TTG AAG CAG 431
Arg Met Arg Glu Arg Pro Ile Gly Asp Leu Val Val Gly Leu Lys Gln
130 135 140

CTT GGT GCA GAT GTT GAT TGT TTC CTT GGC ACT GAC TGC CCA CCT GTT 479
Leu Gly Ala Asp Val Asp Cys Phe Leu Gly Thr Asp Cys Pro Pro Val
145 150 155

CGT GTC AAT GGA ATC GGA GGG CTA CCT GGT GGC AAG GTC AAG CTG TCT 527
Arg Val Asn Gly Ile Gly Gly Leu Pro Gly Gly Lys Val Lys Leu Ser
160 165 170

GGC TCC ATC AGC AGT CAG TAC TTG AGT GCC TTG CTG ATG GCT GCT CCT 575
Gly Ser Ile Ser Ser Gln Tyr Leu Ser Ala Leu Leu Met Ala Ala Pro
175 180 185 190

TTG GCT CTT GGG GAT GTG GAG ATT GAA ATC ATT GAT AAA TTA ATC TCC 623
Leu Ala Leu Gly Asp Val Glu Ile Glu Ile Ile Asp Lys Leu Ile Ser
195 200 205

ATT CCG TAC GTC GAA ATG ACA TTG AGA TTG ATG GAG CGT TTT GGT GTG 671
Ile Pro Tyr Val Glu Met Thr Leu Arg Leu Met Glu Arg Phe Gly Val
210 215 220

AAA GCA GAG CAT TCT GAT AGC TGG GAC AGA TTC TAC ATT AAG GGA GGT 719
Lys Ala Glu His Ser Asp Ser Trp Asp Arg Phe Tyr Ile Lys Gly Gly
225 230 235

SEQ ID N° 2 (suite).

CAA AAA TAC AAG TCC CCT AAA AAT GCC TAT GTT GAA GGT GAT GCC TCA 767
Gln Lys Tyr Lys Ser Pro Lys Asn Ala Tyr Val Glu Gly Asp Ala Ser
240 245 250

AGC GCA AGC TAT TTC TTG GCT GGT GCT GCA ATT ACT GGA GGG ACT GTG 815
Ser Ala Ser Tyr Phe Leu Ala Gly Ala Ala Ile Thr Gly Gly Thr Val
255 260 265 270

ACT GTG GAA GGT TGT GGC ACC ACC AGT TTG CAG GGT GAT GTG AAG TTT 863
Thr Val Glu Gly Cys Gly Thr Thr Ser Leu Gln Gly Asp Val Lys Phe
275 280 285

GCT GAG GTA CTG GAG ATG ATG GGA GCG AAG GTT ACA TGG ACC GAG ACT 911
Ala Glu Val Leu Glu Met Met Gly Ala Lys Val Thr Trp Thr Glu Thr
290 295 300

AGC GTA ACT GTT ACT GGC CCA CCG CGG GAG CCA TTT GGG AGG AAA CAC 959
Ser Val Thr Val Thr Gly Pro Pro Arg Glu Pro Phe Gly Arg Lys His
305 310 315

CTC AAG GCG ATT GAT GTC AAC ATG AAC AAG ATG CCT GAT GTC GCC ATG 1007
Leu Lys Ala Ile Asp Val Asn Met Asn Lys Met Pro Asp Val Ala Met
320 325 330

ACT CTT GCT GTG GTT GCC CTC TTT GCC GAT GGC CCG ACA GCC ATC AGA 1055
Thr Leu Ala Val Val Ala Leu Phe Ala Asp Gly Pro Thr Ala Ile Arg
335 340 345 350

GAC GTG GCT TCC TGG AGA GTA AAG GAG ACC GAG AGG ATG GTT GCG ATC 1103
Asp Val Ala Ser Trp Arg Val Lys Glu Thr Glu Arg Met Val Ala Ile
355 360 365

CGG ACG GAG CTA ACC AAG CTG GGA GCA TCT GTT GAG GAA GGG CCG GAC 1151
Arg Thr Glu Leu Thr Lys Leu Gly Ala Ser Val Glu Glu Gly Pro Asp
370 375 380

TAC TGC ATC ATC ACG CCG CCG GAG AAG CTG AAC GTG ACG GCG ATC GAC 1199
Tyr Cys Ile Ile Thr Pro Pro Glu Lys Leu Asn Val Thr Ala Ile Asp
385 390 395

ACG TAC GAC GAC CAC AGG ATG GCC ATG GCC TTC TCC CTT GCC GCC TGT 1247
Thr Tyr Asp Asp His Arg Met Ala Met Ala Phe Ser Leu Ala Ala Cys
400 405 410

GCC GAG GTC CCC GTC ACC ATC CGG GAC CCT GGG TGC ACC CGG AAG ACC 1295
Ala Glu Val Pro Val Thr Ile Arg Asp Pro Gly Cys Thr Arg Lys Thr
415 420 425 430

TTC CCC GAC TAC TTC GAT GTG CTG AGC ACT TTC GTC AAG AAT 1337
Phe Pro Asp Tyr Phe Asp Val Leu Ser Thr Phe Val Lys Asn
435 440

TAA 1340

SEQ ID N° 3.

Ala Gly Ala Glu Glu Ile Val Leu Gln Pro Ile Lys Glu Ile Ser Gly
1               5                   10                  15

Thr Val Lys Leu Pro Gly Ser Lys Ser Leu Ser Asn Arg Ile Leu Leu
            20                  25                  30

Leu Ala Ala Leu Ser Glu Gly Thr Thr Val Val Asp Asn Leu Leu Asn
        35                  40                  45

Ser Glu Asp Val His Tyr Met Leu Gly Ala Leu Arg Thr Leu Gly Leu
    50                  55                  60

Ser Val Glu Ala Asp Lys Ala Ala Lys Arg Ala Val Val Val Gly Cys
65                  70                  75                  80

Gly Gly Lys Phe Pro Val Glu Asp Ala Lys Glu Glu Val Gln Leu Phe
                85                  90                  95

Leu Gly Asn Ala Gly Thr Ala Met Arg Pro Leu Thr Ala Ala Val Thr
            100                 105                 110

Ala Ala Gly Gly Asn Ala Thr Tyr Val Leu Asp Gly Val Pro Arg Met
        115                 120                 125

Arg Glu Arg Pro Ile Gly Asp Leu Val Val Gly Leu Lys Gln Leu Gly
    130                 135                 140

Ala Asp Val Asp Cys Phe Leu Gly Thr Asp Cys Pro Pro Val Arg Val
145                 150                 155                 160

Asn Gly Ile Gly Gly Leu Pro Gly Gly Lys Val Lys Leu Ser Gly Ser
                165                 170                 175

Ile Ser Ser Gln Tyr Leu Ser Ala Leu Leu Met Ala Ala Pro Leu Ala
            180                 185                 190

Leu Gly Asp Val Glu Ile Glu Ile Ile Asp Lys Leu Ile Ser Ile Pro
        195                 200                 205

Tyr Val Glu Met Thr Leu Arg Leu Met Glu Arg Phe Gly Val Lys Ala
    210                 215                 220

Glu His Ser Asp Ser Trp Asp Arg Phe Tyr Ile Lys Gly Gly Gln Lys
225                 230                 235                 240

Tyr Lys Ser Pro Lys Asn Ala Tyr Val Glu Gly Asp Ala Ser Ser Ala
                245                 250                 255

Ser Tyr Phe Leu Ala Gly Ala Ala Ile Thr Gly Gly Thr Val Thr Val
            260                 265                 270

Glu Gly Cys Gly Thr Thr Ser Leu Gln Gly Asp Val Lys Phe Ala Glu
        275                 280                 285

Val Leu Glu Met Met Gly Ala Lys Val Thr Trp Thr Glu Thr Ser Val
    290                 295                 300

Thr Val Thr Gly Pro Pro Arg Glu Pro Phe Gly Arg Lys His Leu Lys
305                 310                 315                 320

Ala Ile Asp Val Asn Met Asn Lys Met Pro Asp Val Ala Met Thr Leu
                325                 330                 335

Ala Val Val Ala Leu Phe Ala Asp Gly Pro Thr Ala Ile Arg Asp Val
            340                 345                 350

Ala Ser Trp Arg Val Lys Glu Thr Glu Arg Met Val Ala Ile Arg Thr
        355                 360                 365

Glu Leu Thr Lys Leu Gly Ala Ser Val Glu Glu Gly Pro Asp Tyr Cys
    370                 375                 380

Ile Ile Thr Pro Pro Glu Lys Leu Asn Val Thr Ala Ile Asp Thr Tyr
385                 390                 395                 400

Asp Asp His Arg Met Ala Met Ala Phe Ser Leu Ala Ala Cys Ala Glu
                405                 410                 415

Val Pro Val Thr Ile Arg Asp Pro Gly Cys Thr Arg Lys Thr Phe Pro
            420                 425                 430

Asp Tyr Phe Asp Val Leu Ser Thr Phe Val Lys Asn
        435                 440

SEQ ID N° 4.

CCATG GCC GGC GCC GAG GAG ATC GTG CTG CAG CCC ATC AAG GAG ATC 47
Ala Gly Ala Glu Glu Ile Val Leu Gln Pro Ile Lys Glu Ile
1               5                   10

TCC GGC ACC GTC AAG CTG CCG GGG TCC AAG TCG CTT TCC AAC CGG ATC 95
Ser Gly Thr Val Lys Leu Pro Gly Ser Lys Ser Leu Ser Asn Arg Ile
15                  20                  25                  30

CTC CTA CTC GCC GCC CTG TCC GAG GGG ACA ACA GTG GTT GAT AAC CTG 143
Leu Leu Leu Ala Ala Leu Ser Glu Gly Thr Thr Val Val Asp Asn Leu
                35                  40                  45

CTG AAC AGT GAG GAT GTC CAC TAC ATG CTC GGG GCC TTG AGG ACT CTT 191
Leu Asn Ser Glu Asp Val His Tyr Met Leu Gly Ala Leu Arg Thr Leu
            50                  55                  60

GGT CTC TCT GTC GAA GCG GAC AAA GCT GCC AAA AGA GCT GTA GTT GTT 239
Gly Leu Ser Val Glu Ala Asp Lys Ala Ala Lys Arg Ala Val Val Val
        65                  70                  75

GGC TGT GGT GGA AAG TTC CCA GTT GAG GAT GCT AAA GAG GAA GTG CAG 287
Gly Cys Gly Gly Lys Phe Pro Val Glu Asp Ala Lys Glu Glu Val Gln
    80                  85                  90

CTC TTC TTG GGG AAT GCT GGA ATC GCA ATG CGG TCC TTG ACA GCA GCT 335
Leu Phe Leu Gly Asn Ala Gly Ile Ala Met Arg Ser Leu Thr Ala Ala
95                  100                 105                 110

GTT ACT GCT GCT GGT GGA AAT GCA ACT TAC GTG CTT GAT GGA GTA CCA 383
Val Thr Ala Ala Gly Gly Asn Ala Thr Tyr Val Leu Asp Gly Val Pro
                115                 120                 125

AGA ATG AGG GAG AGA CCC ATT GGC GAC TTG GTT GTC GGA TTG AAG CAG 431
Arg Met Arg Glu Arg Pro Ile Gly Asp Leu Val Val Gly Leu Lys Gln
            130                 135                 140

CTT GGT GCA GAT GTT GAT TGT TTC CTT GGC ACT GAC TGC CCA CCT GTT 479
Leu Gly Ala Asp Val Asp Cys Phe Leu Gly Thr Asp Cys Pro Pro Val
        145                 150                 155

CGT GTC AAT GGA ATC GGA GGG CTA CCT GGT GGC AAG GTC AAG CTG TCT 527
Arg Val Asn Gly Ile Gly Gly Leu Pro Gly Gly Lys Val Lys Leu Ser
    160                 165                 170

GGC TCC ATC AGC AGT CAG TAC TTG AGT GCC TTG CTG ATG GCT GCT CCT 575
Gly Ser Ile Ser Ser Gln Tyr Leu Ser Ala Leu Leu Met Ala Ala Pro
175                 180                 185                 190

TTG GCT CTT GGG GAT GTG GAG ATT GAA ATC ATT GAT AAA TTA ATC TCC 623
Leu Ala Leu Gly Asp Val Glu Ile Glu Ile Ile Asp Lys Leu Ile Ser
                195                 200                 205

ATT CCG TAC GTC GAA ATG ACA TTG AGA TTG ATG GAG CGT TTT GGT GTG 671
Ile Pro Tyr Val Glu Met Thr Leu Arg Leu Met Glu Arg Phe Gly Val
            210                 215                 220

AAA GCA GAG CAT TCT GAT AGC TGG GAC AGA TTC TAC ATT AAG GGA GGT 719
Lys Ala Glu His Ser Asp Ser Trp Asp Arg Phe Tyr Ile Lys Gly Gly
        225                 230                 235

SEQ ID N° 4 (suite).

CAA AAA TAC AAG TCC CCT AAA AAT GCC TAT GTT GAA GGT GAT GCC TCA 767
Gln Lys Tyr Lys Ser Pro Lys Asn Ala Tyr Val Glu Gly Asp Ala Ser
240 245 250

AGC GCA AGC TAT TTC TTG GCT GGT GCT GCA ATT ACT GGA GGG ACT GTG 815
Ser Ala Ser Tyr Phe Leu Ala Gly Ala Ala Ile Thr Gly Gly Thr Val
255 260 265 270

ACT GTG GAA GGT TGT GGC ACC ACC AGT TTG CAG GGT GAT GTG AAG TTT 863
Thr Val Glu Gly Cys Gly Thr Thr Ser Leu Gln Gly Asp Val Lys Phe
275 280 285

GCT GAG GTA CTG GAG ATG ATG GGA GCG AAG GTT ACA TGG ACC GAG ACT 911
Ala Glu Val Leu Glu Met Met Gly Ala Lys Val Thr Trp Thr Glu Thr
290 295 300

AGC GTA ACT GTT ACT GGC CCA CCG CGG GAG CCA TTT GGG AGG AAA CAC 959
Ser Val Thr Val Thr Gly Pro Pro Arg Glu Pro Phe Gly Arg Lys His
305 310 315

CTC AAG GCG ATT GAT GTC AAC ATG AAC AAG ATG CCT GAT GTC GCC ATG 1007
Leu Lys Ala Ile Asp Val Asn Met Asn Lys Met Pro Asp Val Ala Met
320 325 330

ACT CTT GCT GTG GTT GCC CTC TTT GCC GAT GGC CCG ACA GCC ATC AGA 1055
Thr Leu Ala Val Val Ala Leu Phe Ala Asp Gly Pro Thr Ala Ile Arg
335 340 345 350

GAC GTG GCT TCC TGG AGA GTA AAG GAG ACC GAG AGG ATG GTT GCG ATC 1103
Asp Val Ala Ser Trp Arg Val Lys Glu Thr Glu Arg Met Val Ala Ile
355 360 365

CGG ACG GAG CTA ACC AAG CTG GGA GCA TCT GTT GAG GAA GGG CCG GAC 1151
Arg Thr Glu Leu Thr Lys Leu Gly Ala Ser Val Glu Glu Gly Pro Asp
370 375 380

TAC TGC ATC ATC ACG CCG CCG GAG AAG CTG AAC GTG ACG GCG ATC GAC 1199
Tyr Cys Ile Ile Thr Pro Pro Glu Lys Leu Asn Val Thr Ala Ile Asp
385 390 395

ACG TAC GAC GAC CAC AGG ATG GCG ATG GCC TTC TCC CTT GCC GCC TGT 1247
Thr Tyr Asp Asp His Arg Met Ala Met Ala Phe Ser Leu Ala Ala Cys
400 405 410

GCC GAG GTC CCC GTC ACC ATC CGG GAC CCT GGG TGC ACC CGG AAG ACC 1295
Ala Glu Val Pro Val Thr Ile Arg Asp Pro Gly Cys Thr Arg Lys Thr
415 420 425 430

TTC CCC GAC TAC TTC GAT GTG CTG AGC ACT TTC GTC AAG AAT 1337
Phe Pro Asp Tyr Phe Asp Val Leu Ser Thr Phe Val Lys Asn
435 440

TAA 1340

SEQ ID N° 5.

Ala Gly Ala Glu Glu Ile Val Leu Gln Pro Ile Lys Glu Ile Ser Gly
1               5                   10                  15

Thr Val Lys Leu Pro Gly Ser Lys Ser Leu Ser Asn Arg Ile Leu Leu
            20                  25                  30

Leu Ala Ala Leu Ser Glu Gly Thr Thr Val Val Asp Asn Leu Leu Asn
        35                  40                  45

Ser Glu Asp Val His Tyr Met Leu Gly Ala Leu Arg Thr Leu Gly Leu
    50                  55                  60

Ser Val Glu Ala Asp Lys Ala Ala Lys Arg Ala Val Val Val Gly Cys
65                  70                  75                  80

Gly Gly Lys Phe Pro Val Glu Asp Ala Lys Glu Glu Val Gln Leu Phe
                85                  90                  95

Leu Gly Asn Ala Gly Ile Ala Met Arg Ser Leu Thr Ala Ala Val Thr
            100                 105                 110

Ala Ala Gly Gly Asn Ala Thr Tyr Val Leu Asp Gly Val Pro Arg Met
        115                 120                 125

Arg Glu Arg Pro Ile Gly Asp Leu Val Val Gly Leu Lys Gln Leu Gly
    130                 135                 140

Ala Asp Val Asp Cys Phe Leu Gly Thr Asp Cys Pro Pro Val Arg Val
145                 150                 155                 160

Asn Gly Ile Gly Gly Leu Pro Gly Gly Lys Val Lys Leu Ser Gly Ser
                165                 170                 175

Ile Ser Ser Gln Tyr Leu Ser Ala Leu Leu Met Ala Ala Pro Leu Ala
            180                 185                 190

Leu Gly Asp Val Glu Ile Glu Ile Ile Asp Lys Leu Ile Ser Ile Pro
        195                 200                 205

Tyr Val Glu Met Thr Leu Arg Leu Met Glu Arg Phe Gly Val Lys Ala
    210                 215                 220

Glu His Ser Asp Ser Trp Asp Arg Phe Tyr Ile Lys Gly Gly Gln Lys
225                 230                 235                 240

Tyr Lys Ser Pro Lys Asn Ala Tyr Val Glu Gly Asp Ala Ser Ser Ala
                245                 250                 255

Ser Tyr Phe Leu Ala Gly Ala Ala Ile Thr Gly Gly Thr Val Thr Val
            260                 265                 270

Glu Gly Cys Gly Thr Thr Ser Leu Gln Gly Asp Val Lys Phe Ala Glu
        275                 280                 285

Val Leu Glu Met Met Gly Ala Lys Val Thr Trp Thr Glu Thr Ser Val
    290                 295                 300

Thr Val Thr Gly Pro Pro Arg Glu Pro Phe Gly Arg Lys His Leu Lys
305                 310                 315                 320

Ala Ile Asp Val Asn Met Asn Lys Met Pro Asp Val Ala Met Thr Leu
                325                 330                 335

Ala Val Val Ala Leu Phe Ala Asp Gly Pro Thr Ala Ile Arg Asp Val
            340                 345                 350

Ala Ser Trp Arg Val Lys Glu Thr Glu Arg Met Val Ala Ile Arg Thr
        355                 360                 365

Glu Leu Thr Lys Leu Gly Ala Ser Val Glu Glu Gly Pro Asp Tyr Cys
    370                 375                 380

Ile Ile Thr Pro Pro Glu Lys Leu Asn Val Thr Ala Ile Asp Thr Tyr
385                 390                 395                 400

Asp Asp His Arg Met Ala Met Ala Phe Ser Leu Ala Ala Cys Ala Glu
                405                 410                 415

Val Pro Val Thr Ile Arg Asp Pro Gly Cys Thr Arg Lys Thr Phe Pro
            420                 425                 430

Asp Tyr Phe Asp Val Leu Ser Thr Phe Val Lys Asn
        435                 440

SEQ ID N° 6.

TGAGGTACGA TTCTTCGATC CTCTTTGATT TTCCTGGAAA TATTTTTTCG GTGATCGTGA 60
AACTACTGGA ATCGCTCGAT AGGTGGTACG AAATTAGGCG AGATTAGTTT CTATTCTTGG 120
CCATTATCTT GTTTCTTCGC CGAATGATCT TCCGTATAAA GATTTTAGGT TAGAGATGAA 180
TCGTATAGCT AGATTTCATC ACCAGATAGT TTCTTTGTCT AGAATCTCTG AAATTCTCGA 240
TAGTTTTCAC ATGTGTAAAT AGATTGTTCT TATTCGGCGA TTGTTGATTA GGGTTTTGAT 300
TTTCTTGATT ATGCGATTGC AATTAGGGAT TTTCTTTGGT TTTGTGTTGA TCTTACGATA 360
CATTCCTGCA ATTGAATACG TATGGATCTA AATCTTGTTA ATTTGTTGAA CAGATCCC 418

SEQ ID N° 7.

CTCAGGCGAA GAACAGGTAT GATTTGTTTG TAATTAGATC AGGGGTTTAG GTCTTTCCAT 60
TACTTTTTAA TGTTTTTTCT GTTACTGTCT CCGCGATCTG ATTTTACGAC AATAGAGTTT 120
CGGGTTTTGT CCCATTCCAG TTTGAAAATA AAGGTCCGTC TTTTAAGTTT GCTGGATCGA 180
TAAACCTGTG AAGATTGAGT CTAGTCGATT TATTGGATGA TCCATTCTTC ATCGTTTTTT 240
TCTTGCTTCG AAGTTCTGTA TAACCAGATT TGTCTGTGTG CGATTGTCAT TACCTAGCCG 300
TGTATCGAGA ACTAGGGTTT TCGAGTCAAT TTTGCCCCTT TTGGTTATAT CTGGTTCGAT 360
AACGATTCAT CTGGATTAGG GTTTTAAGTG GTGACGTTTA GTATTCCAAT TTCTTCAAAA 420
TTTAGTTATG GATAATGAAA ATCCCCAATT GACTGTTCAA TTTCTTGTTA AATGCGCAGA 480
TCCCGGGATC TGCG 494

## Revendications

1. Séquence ADN isolée pouvant servir de zone de régulation dans un gène chimère utilisable pour la transformation des plantes et permettant l'expression du produit de traduction du gène chimère en particulier dans les régions de la plante en croissance rapide, **caractérisée en ce qu'**elle comprend au moins un intron correspondant au premier intron (intron 1) de la région 5' non-codante d'un gène d'histone végétale de type H3.3.

2. Séquence ADN selon la revendication 1, **caractérisée en ce que** l'intron 1 de la région 5' non-codante d'un gène d'histone végétale provient d'une plante dicotylédone.

3. Séquence ADN selon la revendication 2, **caractérisée en ce que** la plante dicotylédone est *Arabidopsis thaliana*.

4. Séquence ADN selon la revendication 3, **caractérisée en ce qu'**elle est sélectionnée parmi le groupe consistant en SEQ ID NO:6 et SEQ ID NO:7

5. Séquence ADN selon la revendication 1, **caractérisée en ce que** l'intron 1 de la région 5' non-codante d'un gène d'histone végétale provient d'une plante monocotylédone.

6. Séquence ADN selon la revendication 5, **caractérisée en ce que** la plante monocotylédone est *Zea mays*.

7. Séquence ADN selon l'une des revendications 1 à 6, **caractérisée en ce que** l'intron 1 est orienté, dans le sens de la transcription du gène chimère, de façon directe ou inversée relativement à son orientation initiale dans le gène dont il est issu.

8. Séquence ADN selon l'une des revendications 1 à 7, **caractérisée en ce qu'**elle comprend deux introns 1, identiques ou différents, associés.

9. Gène chimère pour la transformation des plantes comprenant au moins, dans le sens de la transcription, une zone promotrice, une zone de régulation, et une séquence codant pour une enzyme de tolérance herbicide, **caractérisé en ce que** la zone de régulation comprend en outre une séquence ADN selon l'une des revendications 1 à 8.

10. Gène chimère selon la revendication 9, **caractérisé en ce que** la zone promotrice provient d'un promoteur de gène d'histone végétale.

11. Gène chimère selon l'une des revendications 9 et ou10, **caractérisé en ce que** la zone promotrice provient du même gène d'histone végétale que l'intron 1.

12. Gène chimère selon l'une des revendications 9 à 11, **caractérisé en ce que** la zone promotrice comprend un promoteur d'histone végétale dupliqué.

13. Gène chimère selon l'une des revendications 9 à 12, **caractérisé en ce que** la zone promotrice contient au moins un promoteur d'un gène d'histone végétale associé à un promoteur différent issu d'un gène pouvant s'exprimer naturellement dans les plantes.

14. Gène chimère selon l'une des revendications 9 à 13, **caractérisé en ce que** la séquence codant pour une enzyme de tolérance herbicide est fusionnée à une séquence ADN codant pour une zone peptide de transit permettant l'accumulation de ladite enzyme de tolérance herbicide dans un compartiment subcellulaire.

15. Gène chimère selon la revendication 14, **caractérisé en ce que** la zone peptide de transit permet l'accumulation de ladite enzyme de tolérance herbicide dans le compartiment plastidial.

16. Gène chimère selon la revendication 15, **caractérisé en ce que** la zone peptide de transit comprend, dans le sens de la transcription, au moins un peptide de transit d'un gène végétal codant pour une enzyme à localisation plastidiale, éventuellement une partie de séquence de la partie mature N-terminale d'un gène végétal codant pour une enzyme à localisation plastidiale, puis éventuellement un second peptide de transit d'un gène végétal codant pour une enzyme à localisation plastidiale.

17. Gène chimère selon l'une des revendications 9 à 16, **caractérisé en ce que** la séquence codant pour une enzyme de tolérance herbicide code pour une enzyme active vis à vis d'un herbicide dont la cible est l'EPSPS.

18. Gène chimère selon la revendication 17, **caractérisé en ce que** l'herbicide dont la cible est l'EPSPS est le glyphosate.

19. Vecteur pour la transformation des plantes, **caractérisé en ce qu'**il comprend un gène chimère selon l'une des revendications 9 à 18.

20. Souche d'*Agrobacterium* sp., **caractérisée en ce qu'**elle contient un vecteur selon la revendication 19.

21. Cellule végétale transformée, **caractérisée en ce qu'**elle contient un gène chimère selon l'une des revendications 9 à 18.

22. Plante ou partie de plante transformée obtenue à partir d'une cellule selon la revendication 21.

23. Procédé de construction d'un gène chimère selon l'une des revendications 9 à 18, **caractérisé en ce qu'**on isole respectivement une séquence ADN selon l'une des revendications 1 à 8, une zone promotrice, une zone peptide de transit, ainsi qu'au moins un transgène, et qu'ensuite on les assemble dans le sens de la transcription du transgène.

## Claims

1. Isolated DNA sequence capable of serving as regulatory zone in a chimeric gene which can be used for the transformation of plants and allowing the expression of the product of translation of the chimeric gene in particular in the regions of the plant undergoing rapid growth, **characterized in that** it comprises at least one intron corresponding to the first intron (intron 1) of the noncoding 5' region of a plant histone gene of H3.3 type.

2. DNA sequence according to Claim 1, **characterized in that** intron 1 of the noncoding 5' region of a plant histone gene comes from a dicotyledonous plant.

3. DNA sequence according to Claim 2, **characterized in that** the dicotyledonous plant is *Arabidopsis thaliana*.

4. DNA sequence according to Claim 3, **characterized in that** it is chosen from the group consisting of SEQ ID NO. 6 and SEQ ID NO. 7.

5. DNA sequence according to Claim 1, **characterized in that** intron 1 of the noncoding 5' region of a plant histone gene comes from a monocotyledonous plant.

6. DNA sequence according to Claim 5, **characterized in that** the monocotyledonous plant is *Zea mays.*

7. DNA sequence according to one of Claims 1 to 6, **characterized in that** intron 1 is oriented, in the direction of transcription of the chimeric gene, in a direct or reversed manner relative to its initial orientation in the gene from which it is derived.

8. DNA sequence according to one of Claims 1 to 7, **characterized in that** it comprises two introns 1, identical or different, which are combined.

9. Chimeric gene for the transformation of plants comprising at least, in the direction of transcription, a promoter zone, a regulatory zone and a sequence encoding a herbicide tolerance enzyme, **characterized in that** the regulatory zone comprises a DNA sequence according to one of Claims 1 to 8.

10. Chimeric gene according to Claim 9, **characterized in that** the promoter zone comes from a promoter of a plant histone gene.

11. Chimeric gene according to either of Claims 9 and/or 10, **characterized in that** the promoter zone comes from the same plant histone gene as intron 1.

12. Chimeric gene according to one of Claims 9 to 11, **characterized in that** the promoter zone comprises a duplicated plant histone promoter.

13. Chimeric gene according to one of Claims 9 to 12, **characterized in that** the promoter zone contains at least one promoter of a plant histone gene combined with a different promoter derived from a gene which can be naturally expressed in plants.

14. Chimeric gene according to one of Claims 9 to 13, **characterized in that** the sequence encoding a herbicide tolerance enzyme is fused with a DNA sequence encoding a transit peptide zone allowing the accumulation of the said herbicide tolerance enzyme in a subcellular compartment.

15. Chimeric gene according to Claim 14, **characterized in that** the transit peptide zone allows the accumulation of the said herbicide tolerance enzyme in the plastid compartment.

16. Chimeric gene according to Claim 15, **characterized in that** the transit peptide zone comprises, in the direction of transcription, at least one transit peptide of a plant gene encoding a plastid-localized enzyme, optionally a portion of sequence of the N-terminal mature part of a plant gene encoding a plastid-localized enzyme, and then optionally a second transit peptide of a plant gene encoding a plastid-localized enzyme.

17. Chimeric gene according to one of Claims 9 to 16, **characterized in that** the sequence encoding a herbicide tolerance enzyme encodes an enzyme which is active towards a herbicide whose target is EPSPS.

18. Chimeric gene according to Claim 17, **characterized in that** the herbicide whose target is EPSPS is glyphosate.

19. Vector for the transformation of plants, **characterized in that** it comprises a chimeric gene according to one of Claims 9 to 18.

20. Strain of *Agrobacterium* sp., **characterized in that** it contains a vector according to Claim 19.

21. Transformed plant cell, **characterized in that** it contains a chimeric gene according to one of Claims 9 to 18.

22. Transformed plant or plant portion obtained from a cell according to Claim 21.

23. Process for the construction of a chimeric gene according to one of Claims 9 to 18, **characterized in that** there are respectively isolated a DNA sequence according to one of claims 1 to 8, a promoter zone, a transit peptide zone, as well as at least one transgene, and **in that** they are then assembled in the direction of transcription of the transgene.

## Patentansprüche

1. Isolierte DNA-Sequenz, die als Regulationsregion in einem Chimären Gen dienen kann, das für die Transformation von Pflanzen brauchbar ist, und die die Expression des Translationsprodukts des chimären Gens insbesondere in den schnell wachsenden Bereichen der Pflanze ermöglicht, **dadurch gekennzeichnet, daß** sie mindestens ein Intron aufweist, das dem ersten Intron (Intron 1) des nicht-codierenden 5'-Bereichs eines pflanzlichen Histon-Gens vom Typ H3.3 entspricht.

2. DNA-Sequenz nach Anspruch 1, **dadurch gekennzeichnet, daß** das Intron 1 des nicht-codierenden 5'-Bereichs eines pflanzlichen Histon-Gens von einer dikotylen Pflanze stammt.

3. DNA-Sequenz nach Anspruch 2, **dadurch gekennzeichnet, daß** es sich bei der dikotylen Pflanze um *Arabidopsis thaliana* handelt.

4. DNA-Sequenz nach Anspruch 3, **dadurch gekennzeichnet, daß** sie unter SEQ ID NO:6 und SEQ ID NO:7 ausgewählt ist.

5. DNA-Sequenz nach Anspruch 1, **dadurch gekennzeichnet, daß** das Intron 1 des nicht-codierenden 5'-Bereichs eines pflanzlichen Histon-Gens von einer monokotylen Pflanze stammt.

6. DNA-Sequenz nach Anspruch 5, **dadurch gekennzeichnet, daß** es sich bei der monokotylen Pflanze um *Zea mays* handelt.

7. DNA-Sequenz nach einem der Ansprüche 1 bis 6, **dadurch gekennzeichnet, daß** das Intron 1, in Transkriptionsrichtung des Chimären Gens, entweder in dieser Richtung oder in umgekehrter Richtung orientiert ist, bezogen auf seine ursprüngliche Orientierung in dem Gen, aus dem es stammt.

8. DNA-Sequenz nach einem der Ansprüche 1 bis 7, **dadurch gekennzeichnet, daß** sie zwei kombinierte Introns 1 aufweist, die gleich oder verschieden sind.

9. Chimäres Gen für die Transformation von Pflanzen, das in Transkriptionsrichtung mindestens eine Promotorregion, eine Regulationsregion und eine Sequenz, die ein Herbizidtoleranz-Enzym codiert, umfaßt, **dadurch gekennzeichnet, daß** die Regulationsregion außerdem eine DNA-Sequenz nach einem der Ansprüche 1 bis 8 umfaßt.

10. Chimäres Gen nach Anspruch 9, **dadurch gekennzeichnet, daß** die Promotorregion von einem Promotor eines pflanzlichen Histon-Gens stammt.

11. Chimäres Gen nach Anspruch 9 und/oder 10, **dadurch gekennzeichnet, daß** die Promotorregion vom gleichen pflanzlichen Histon-Gen wie das Intron 1 stammt.

12. Chimäres Gen nach einem der Ansprüche 9 bis 11, **dadurch gekennzeichnet, daß** die Promotorregion einen verdoppelten Promotor eines pflanzlichen Histons umfaßt.

13. Chimäres Gen nach einem der Ansprüche 9 bis 12, **dadurch gekennzeichnet, daß** die Promotorregion mindestens einen Promotor eines pflanzlichen Histon-Gens enthält, der mit einem davon verschiedenen Promotor kombiniert ist, der von einem Gen stammt, das natürlich in Pflanzen exprimiert werden kann.

14. Chimäres Gen nach einem der Ansprüche 9 bis 13, **dadurch gekennzeichnet, daß** die Sequenz, die ein Herbizidtoleranz-Enzym codiert, mit einer DNA-Sequenz fusioniert ist, die einen Signalpeptidbereich codiert, der die Anreicherung des Herbizidtoleranz-Enzyms in einem subzellulären Kompartiment ermöglicht.

15. Chimäres Gen nach Anspruch 14, **dadurch gekennzeichnet, daß** der Signalpeptidbereich die Anreicherung des Herbizidtoleranz-Enzyms in dem aus den Plastiden bestehenden Kompartiment ermöglicht.

16. Chimäres Gen nach Anspruch 15, **dadurch gekennzeichnet, daß** der Signalpeptidbereich in Transkriptionsrichtung mindestens umfaßt: ein Signalpeptid eines pflanzlichen Gens, das ein in den Plastiden lokalisiertes Enzym codiert, gegebenenfalls einen Teil der Sequenz des N-terminalen reifen Teils eines pflanzlichen Gens, das ein in den Plastiden lokalisiertes Enzym codiert, und gegebenenfalls ein zweites Signalpeptid eines pflanzliche Gens, das ein in den Plastiden lokalisiertes Enzym codiert.

17. Chimäres Gen nach einem der Ansprüche 9 bis 16, **dadurch gekennzeichnet, daß** die Sequenz, die ein Herbizidtoleranz-Enzym codiert, ein Enzym codiert, das gegenüber einem Herbizid wirksam ist, das gegen EPSPS gerichtet ist.

18. Chimäres Gen nach Anspruch 17, **dadurch gekennzeichnet, daß** es sich bei dem Herbizid, das gegen EPSPS gerichtet ist, um Glyphosat handelt.

19. Vektor für die Transformation von Pflanzen, **dadurch gekennzeichnet, daß** er ein chimäres Gen nach einem der Ansprüche 9 bis 18 enthält.

20. Stamm von *Agrobacterium* sp., **dadurch gekennzeichnet, daß** er einen Vektor nach Anspruch 19 enthält.

21. Transformierte pflanzliche Zelle, **dadurch gekennzeichnet, daß** sie ein chimäres Gen nach einem der Ansprüche 9 bis 18 enthält.

22. Transformierte Pflanze oder Teil einer transformierten Pflanze, die/das aus einer Zelle nach Anspruch 21 erhältlich ist.

23. Verfahren zur Konstruktion eines Chimären Gens nach einem der Ansprüche 9 bis 18, **dadurch gekennzeichnet, daß** eine DNA-Sequenz nach einem der Ansprüche 1 bis 8, eine Promotorregion, ein Signalpeptidbereich sowie mindestens ein Transgen isoliert werden und daß anschließend diese Bestandteile in Transkriptionsrichtung des Transgens zusammengefügt werden.
